# FASCICULE DE BREVET EUROPEEN

(11) **EP 0 785 432 B1**
(45) Date de publication et mention de la délivrance du brevet: **13.09.2000**
(21) Numéro de dépôt: 96430018.0
(22) Date de dépôt: 16.12.1996
(51) Int. Cl.: G01N 33/531, G01N 33/74, G01N 33/78, C12N 5/20, C07K 16/26, C12P 21/08

(54) **Procédé pour l'identification ou le dosage immunologique d'antigènes polypeptidiques**
Verfahren zur immunologischen Identifizierung oder Bestimmung von Polypeptid-Antigenen
Method for the immunological identification or determination of polypeptide antigens

(30) Priorité: 18.01.1996 FR 9600894
(43) Date de publication de la demande: 23.07.1997
(73) Titulaire: Société Anonyme dite: IMMUNOTECH S.A., F-13276 Marseille Cédex 9 (FR)
(72) Inventeur: Jean, Fréderic, 72 rue Henri Tomasi, 13009 Marseille (FR); Kertesz, Gilles, 13005 Marseille (FR); Bourcier, Béatrice, 13005 Marseille (FR)
(74) Mandataire: Rinuy, Santarelli

(56) Documents cités:
- EP-A- 0 265 327
- EP-A- 0 280 561
- FR-A- 2 658 301
- GB-A- 1 505 400
- GB-A- 2 090 598
- US-A- 4 415 546
- NATURE, vol. 210, no. 5035, 30 Avril 1966, LONDON GB, pages 536-537, XP002014540 J. LENARD ET AL.: "Succinylation of gamma globulin."

## Description

La présente invention concerne un nouveau procédé pour l'identification ou le dosage immunologique d'antigènes polypeptidiques.

FR-A-2 658 301 décrit un procédé d'identification ou de dosage de l'ensemble des protéines pontées entre elles par des dialdéhydes provenant de l'oxydation in vivo des acides gras polyinsaturés lors de la dégénérescence cellulaire. Ce procédé met en jeu un anticorps monoclonal spécifique non pas d'une protéine particulière, mais spécifique de la modification elle-même.

Parmi les antigènes peptidiques et protéiques, certains peuvent avoir un caractère faiblement immunogénique. Autrement dit, lorsqu'ils sont injectés à certains animaux par les méthodes d'immunisation classiques, il est rare d'observer une réponse anticorps contre ces antigènes. C'est par exemple le cas pour les hormones peptidiques humaines ACTH (hormone corticotrope) et PTH (parathormone), lorsque l'on immunise par exemple des souris, des rats, ou des lapins. Les quelques anticorps dirigés contre ces molécules, possédant les qualités requises pour une application dans un dosage immunologique, ont été obtenus le plus souvent avec beaucoup de difficulté par immunisation avec la molécule complète ou un fragment, couplé à une protéine porteuse par la méthode à la carbodiimide ou la méthode à la glutaraldéhyde. Ces deux exemples ne sont évidemment pas limitatifs, et de nombreux autres antigènes protéiques solubles, ou membranaires à la surface de cellules, peuvent également être considérés comme peu immunogéniques.

La faible immunogénicité va dépendre à la fois des propriétés physicochimiques de l'antigène et des caractéristiques du système immunitaire de l'animal immunisé. Deux causes principales peuvent entraîner une absence de réponse anticorps chez un animal immunisé par un antigène peptidique. La première est une mauvaise stabilité de la molécule in vivo: la demi-vie de l'antigène après une injection chez l'animal sera alors trop courte pour stimuler une réponse anticorps. La deuxième est un "trou" dans le répertoire anticorps de l'animal immunisé. Ce "trou" dans le répertoire peut être lié, en particulier, à une forte homologie de structure entre l'immunogène protéique et la protéine endogène correspondante de l'animal immunisé. Cette ressemblance conduira le système immunitaire à ne pas reconnaître l'immunogène comme étranger au soi, et donc à ne pas produire d'anticorps en réponse à l'injection du peptide. Ceci est évidemment particulièrement vrai dans le cas où l'on veut immuniser un animal avec une protéine strictement identique à la sienne.

Ces deux explications peuvent être illustrées par le cas de l'ACTH humaine. La demi-vie de cette hormone injectée par voie intraveineuse chez le rat, est de l'ordre de 5 minutes; c'est donc une molécule très instable. Sur les 39 acides aminés qui la composent, 3 résidus seulement sont différents entre les protéines humaine et murine ; c'est donc un antigène très bien conservé entre ces deux espèces. Lorsqu'il est injecté à une souris, cet antigène ne conduit donc que rarement à la production d'anticorps.

En dépit de leur caractère faiblement immunogénique, il serait souhaitable de disposer d'un procédé facilitant l'obtention d'anticorps utilisables par exemple à la détection ou au dosage de ces antigènes. Ce procédé devrait conduire à une plus grande variété d'anticorps, qui devrait permettre de développer notamment des systèmes de dosage et de détection plus performants que ceux qui utilisent actuellement les rares anticorps obtenus par les méthodes classiques. En particulier, dans le cas de la plupart des dosages immunologiques de l'ACTH et de la PTH humaines, les sensibilités requises ne sont atteintes que pour des temps d'incubations très longs (souvent plus de 12 heures), et il y aurait donc intérêt à produire, à l'aide d'un tel procédé, des anticorps permettant de développer des dosages beaucoup plus rapides.

Par ailleurs, on a vu ci-dessus que le caractère faiblement immunogénique d'un antigène peptidique pouvait être lié à une mauvaise stabilité in-vivo. Cette mauvaise stabilité peut se traduire in-vitro par une dégradation rapide de l'antigène dans l'échantillon à doser. Cette dégradation peut donc conduire à une sous estimation de la concentration réelle de l'antigène. Il y aurait donc grand intérêt à disposer d'un procédé qui permette de stabiliser de tels antigènes pour faciliter la manipulation des échantillons et éviter la dégradation.

C'est pourquoi la présente invention a pour objet un nouveau procédé d'identification ou de dosage d'un antigène polypeptidique X particulier dans lequel on procède à une détermination immunologique de la présence dudit antigène polypeptidique X dans un échantillon, caractérisé en ce que l'on traite in vitro l'échantillon à l'aide d'au moins un agent réactif
- sur une fonction epsilon -NH₂ d'un reste lysine ou
- sur une fonction alpha -NH₂ d'un peptide,
pour effectuer le couplage d'un reste dudit agent réactif sur ledit antigène polypeptidique X et ainsi obtenir un antigène polypeptidique X modifié, (antigène polypeptidique Xm),
puis procède à la détermination immunologique de la présence dudit antigène polypeptidique X dans ledit échantillon à l'aide d'au moins un anticorps spécifique dudit antigène polypeptidique Xm et non spécifique de la modification elle-même, produit par immunisation contre un antigène polypeptidique Y identique à l'antigène polypeptidique Xm ou à un fragment antigénique de cet antigène Xm.

Autrement dit, pour produire et utiliser des anticorps pour la détection immunologique d'antigènes peptidiques faiblement immunogéniques, la demanderesse a mis au point une méthode dont le résultat est la modification de la structure de l'antigène. La demanderesse a découvert de manière suprenante que cette modification de structure permettait d'une part de stimuler au cours des immunisations de nouvelles cellules capables de sécréter des anticorps contre cet antigène modifié, et d'échapper ainsi à l'incapacité de l'animal immunisé à produire des anticorps contre l'antigène naturel, et d'autre part d'améliorer dans certains cas la stabilité de l'antigène, en particulier en lui conférant une meilleure résistance aux enzymes protéolytiques.

En effet, l'absence de réponse anticorps chez un animal immunisé n'est pas obligatoirement liée à une identité stricte entre l'antigène polypeptidique et la protéine endogène de l'animal, mais plus généralement à une incapacité génétique à produire des anticorps contre cet antigène, ou à un phénomène de tolérance qui va se traduire par la suppression de cette capacité à produire ces anticorps. L'important dans le présent procédé est donc de modifier l'antigène pour le rendre différent et échapper ainsi à cette incapacité génétique ou à cette suppression.

Cette méthode repose sur une modification chimique de l'antigène polypeptidique naturel X pour obtenir une antigène polypeptidique Xm. Un antigène polypeptidique Y qui est identique à l'antigène peptidique Xm, ou correspond à un fragment de cet antigène sera utilisé au cours des immunisations, dans la mesure où ce fragment correspond à au moins la taille d'un épitope (4 acides aminés) de l'antigène Xm et porte au moins un groupement modifié chimiquement.

La modification chimique de certains acides aminés induit donc un changement de structure du polypeptide faisant apparaître des déterminants antigéniques différents et permet dans certains cas de protéger ou même supprimer les sites de clivage des enzymes protéolytiques, responsables de la dégradation de l'antigène in-vivo et dans les échantillons biologiques, notamment dans les cas où la modification intervient au voisinage de ces sites.

Dans le cadre de la modification chimique de l'échantillon biologique avant le dosage, le changement de structure de certains enzymes protéolytiques peut entraîner une perte d'activité de ces enzymes, ce qui peut se traduire également par une augmentation de la stabilité de l'antigène dans l'échantillon modifié.

Les immunisations avec l'antigène Y conduisent donc à la production d'anticorps dirigés contre l'antigène polypeptidique Xm, qui sont ensuite utilisés par exemple pour doser ou détecter l'antigène X dans un échantillon auquel on fait subir la même modification.

Par "antigène polypeptidique X", l'on entend un polypeptide constitué d'au moins 10 acides aminés, de préférence au moins 25 acides aminés, et par "fragment" d'un antigène polypeptidique l'on entend un peptide constitué d'au moins 4 acides aminés et de préférence de moins de 80% de la totalité des acides aminés de l'antigène polypeptidique complet.

Par "antigène polypeptidique X modifié" Xm, l'on entend qu'une réaction de modification chimique a été réalisée sur l'antigène X à l'aide d'un ou plusieurs agents chimiques réactifs sur un ou plusieurs groupements -NH₂ d'un ou de plusieurs acides aminés comme défini ci-dessus.

Pour modifier ces groupements -NH₂ on peut utiliser un ou plusieurs agents réactifs spécifiques.

Les conditions expérimentales utilisées peuvent favoriser la modification soit de la majorité des groupements -NH₂ réactifs, soit la modification d'une partie seulement de ces groupements, en jouant en particulier sur les quantités de réactifs utilisées, comme le sait l'homme de l'art.

La modification chimique d'un groupement -NH₂ réactif de l'antigène par couplage d'un reste de l'agent réactif utilisé doit être suffisamment petite pour obtenir des anticorps spécifiques de l'antigène polypeptidique modifié et non des anticorps spécifiques de la modification. Le reste couplé devrait avoir de préférence un poids moléculaire inférieur à 400 daltons et notamment inférieur à 200 daltons. On peut contrôler la spécificité de l'anticorps vis à vis de l'antigène en vérifiant que les anticorps ne reconnaissent pas d'autres antigènes polypeptidiques ayant subi la même modification.

Dans une mise en oeuvre préférentielle du procédé ci-dessus décrit, l'antigène polypeptidique Y utilisé dans les immunisations pour préparer des anticorps peut être obtenu comme suit :

L'antigène polypeptidique purifié complet, naturel X, ou un fragment de cet antigène, est modifié par le ou les agents réactifs sélectionnés.

L'antigène polypeptidique Y peut également être synthétique, ou semi synthétique. On peut en particulier préparer par synthèse un polypeptide ayant la séquence en acides aminés du polypeptide X ou d'un fragment X et le modifier en le soumettant à l'action de l'agent, ou des agents réactifs choisis.

L'antigène Y est ensuite injecté directement, ou après couplage à une protéine porteuse, à un animal pour immunisation et obtention d'anticorps monoclonaux ou polyclonaux, suivant les méthodes connues de l'art antérieur. Après une modification préalable de l'échantillon par le, ou les réactifs sélectionnés mentionnés ci-dessus, ces anticorps seront alors utilisés suivant les méthodes connues de l'art antérieur, pour détecter ou doser l'antigène modifié.

Dans le cas où les anticorps produits contre l'antigène Y reconnaissent également l'antigène naturel X non modifié avec une affinité suffisante, le dosage, ou la détection de l'antigène polypeptidique X, peut être effectué sur des échantillons non modifiés.

Le réactif de modification peut être avantageusement choisi pour son efficacité (pour modifier les groupements -NH₂ portés par l'antigène, il doit également modifier les nombreux groupements -NH₂ réactifs portés par les autres protéines présentes dans l'échantillon), pour la rapidité de la réaction de modification, pour la simplicité de la mise en oeuvre de la réaction et pour sa compatibilité avec la réalisation ultérieure d'un dosage immunologique, et enfin pour sa stabilité et sa disponibilité à un moindre coût.

Comme réactifs de modification des groupements epsilon -NH₂ des lysines et alpha -NH₂ des polypeptides, on retiendra préférentiellement les réactifs d'acylation comme les anhydrides d'acides carboxyliques tels que l'anhydride succinique, l'anhydride acétique, ou l'anhydride citraconique, ou les esters activés tels que les dérivés de N-hydroxysuccinimidyl ester.

En particulier avec l'anhydride succinique, la réaction d'acylation est rapide et efficace (on peut modifier pratiquement instantanément la quasi totalité des groupements -NH₂ réactifs dans un échantillon biologique); le nombre de charges portées par la protéine est conservé puisque les groupements -NH₂ sont remplacés par des groupements - COOH, ce qui favorise des interactions antigène-anticorps de haute affinité.

Comme exemple d'antigène, on peut citer l'ACTH, ou la PTH humaine, ou leurs fragments biologiquement actifs, par exemple le fragment 1-24 de l'ACTH, et de manière générale tout antigène polypeptidique soluble ou membranaire comportant des groupements -NH₂ réactifs susceptibles d'être modifiés chimiquement. Le procédé selon l'invention est bien sûr particulièrement intéressant pour les antigènes peu immunogéniques.

La présente invention a également pour objet les hybridomes obtenus selon les méthodes classiques de l'art antérieur, en utilisant comme antigène l'antigène Y défini ci-dessus, lesdits hybridomes permettant la production d'anticorps monoclonaux ou polyclonaux, et notamment un hybridome produisant des anticorps monoclonaux anti-ACTH acylée par l'anhydride succinique, choisi parmi les hybridomes déposés à la Collection Nationale de Culture de Microorganismes le 12 décembre 1995 sous les numéros I-1648, I-1649 et I-1650.

La présente invention a encore pour objet un anticorps monoclonal produit par un hybridome tel que défini ci-dessus.

L'échantillon peut être par exemple un fluide biologique, tel que le sang, le sérum, l'urine, la sueur, les larmes, etc., ou encore un prélèvement de cellules ou de tissus.

La méthode de dosage ou de détection ci-dessus peut utiliser un ou plusieurs anticorps monoclonaux ou polyclonaux et peut être par exemple : un dosage radioimmunologique par compétition, ou radioimmunométrique utilisant un traceur radioactif, luminescent, fluorescent ou enzymatique, ou un dosage immunoenzymométrique. Elle peut être aussi un dosage immunologique par agglutination de particules. Elle peut être encore une détection immunohistochimique sur un prélèvement de tissu, ou une analyse par cytométrie de flux d'un antigène cellulaire, réalisée sur une suspension cellulaire.

Les exemples qui suivent illustrent la présente invention.

### Exemple 1 : Synthèse d'ACTH succinylée.

A 1mg d'ACTH 1-39 de synthèse en solution dans 1 ml de tampon Hepes 0,2M, pH 8,2, on ajoute 180 µl d'anhydride succinique en solution à 3mg/ml dans du diméthyl formamide. Après une heure d'incubation à 20-25°C, le mélange réactionnel est acidifié par addition de 4 ml d'acide trifluoroacétique (TFA) à 1 % dans l'eau. L'ACTH succinylée est ensuite purifiée par chromatographie liquide haute performance en phase reverse (CLHP-PR), à l'aide d'une colonne C18 micro-bondapack ® (Waters, USA) éluée par un gradient d'acétonitrile dans une solution de TFA à 0,05 % dans l'eau.

### Exemple 2 : Préparation des immunogènes ACTH

L'ACTH succinylée est couplée d'une part à la sérum albumine bovine (BSA) et d'autre part à la thyroglobuline porcine (TG) par la méthode à la carbodiimide décrite par Goodfriend dans Science 144, 1344 (1964). A 500 µg d'ACTH succinylée en solution dans 0.5 ml d'eau on ajoute successivement : 1.25 mg de chlorhydrate de 1-éthyl-3-(3-dimethylaminopropyl) carbodiimide (EDC) en solution dans 10 µl d'eau, et 1,5 mg de BSA ou de TG en solution dans 500 µl d'un tampon Mes (acide 2-(N-morpholino) éthane sulfonique) 0,1 M, pH 5,5. Après 4 heures d'incubation à 20-25°C, l'immunogène est purifié par tamisage moléculaire sur une colonne Superdex 200® 16/60 (Pharmacia, Suède) éluée en tampon phosphate de sodium 10 mM, NaCl 150mM, pH : 7,2 (PBS), à un débit de 1 ml/minute.

### Exemple 3 : Immunisation et production des anticorps monoclonaux anti ACTH.

Des souris Balb/c reçoivent des injections mensuelles de 500 µl d'émulsion à volumes égaux de 25 µg d'immunogène obtenu à l'exemple 2 en solution dans du tampon phosphate (PBS) avec de l'adjuvant de Freund. La première injection est réalisée en adjuvant complet et les suivantes en adjuvant incomplet. La moitié du volume est injectée par voie intrapéritonéale et l'autre moitié par voie sous cutanée. Pour chaque souris, 3 à 4 immunisations avec de l'ACTH succinylée couplée à de la BSA sont suivies par 4 à 5 immunisations avec de l'ACTH succinylée couplée avec de la TG. Des prélèvements de sérum sont réalisés 10 jours après chaque immunisation, pour suivre le taux en anticorps anti-ACTH succinylée. Cinq jours avant le prélèvement de leur rate, les animaux sélectionnés pour réaliser une hybridation lymphocytaire reçoivent 50 µl par voie intraveineuse, et 100 µl par voie intra-péritonéale, d'une solution à 1 mg/ml d'ACTH succinylée couplée à de la TG en tampon PBS. Trois hybridations lymphocytaires avec des cellules de myélome X63 AG3 réalisées à partir de trois animaux différents, ont permis d'obtenir 40 hybridomes sécrétant des anticorps monoclonaux anti-ACTH succinylée. Parmi ces hybridomes, après criblage des anticorps dans les surnageants de culture pour leur capacité à reconnaître l'ACTH succinylée marquée à l'Iode 125, et l'ACTH succinylée immobilisée dans les puits d'une plaque de microtitration, on retient les hybridomes IMMU 148, IMMU 299, et IMMU 314 qui ont été déposés à la Collection Nationale de Culture de Microorganismes à Paris, le 12 décembre 1995, sous les Numéros I - 1648; I - 1649, et I - 1650.

Les anticorps monoclonaux utilisés ci-dessus sont d'isotype IgG1 pour IMMU148, et IgA pour IMMU299 et IMMU314. Leur constante de dissociation pour l'ACTH succinylée est respectivement de 1,9 x 10⁻¹⁰M, 1,3 x 10⁻¹¹M, et 3,4 x 10⁻¹⁰M. Ils ont la propriété de pouvoir se lier simultanément et sans compétition à l'ACTH succinylée.

### Exemple 4 : Modification de l'ACTH en solution dans un échantillon plasmatique et dans un standard d'ACTH.

250 µl d'échantillon plasmatique sont déposés dans un tube contenant 6,15 mg d'anhydride succinique et 4 mg de rouge de phénol lyophilisés. 125 µl d'une solution alcaline composée de KOH 0,42 M et de KH₂P0₄ 0,166 M à pH : 6,7 sont ensuite distribués dans chaque tube. La réaction de succinylation est pratiquement immédiate, et se traduit par la modification du colorant de la couleur rouge à la couleur jaune.

On opère de même avec des solutions standards contenant des quantités connues d'ACTH.

### Exemple 5 : Dosage de l'ACTH modifiée

Après avoir subi le traitement par l'anhydride succinique décrit ci-dessus, 300 µl d'échantillon plasmatique, ou de solution standard contenant une quantité connue d'ACTH dans du sérum humain, sont déposés dans un tube recouvert de deux anticorps monoclonaux IMMU 148 et IMMU 314 anti-ACTH modifiée capables de se lier simultanément à l'ACTH succinylée. Les tubes sont alors incubés pendant 1 heure à 20-25°C sous agitation constante. Après avoir été vidé de son contenu par aspiration, chaque tube est lavé par 2 ml d'une solution de NaCl à 9 g/l dans l'eau contenant 0,005 % de Tween 20. 100 µl d'une solution contenant 250000 cpm d'un troisième anticorps monoclonal IMMU 299 marqué à l'iode 125, capable de se lier à l'ACTH succinylée donc simultanément aux deux autres, dans un tampon PBS contenant 0,1 % de BSA, sont distribués dans chaque tube. Après 3 heures d'incubation à 20-25°C sous agitation constante, les tubes sont à nouveau vidés de leur contenu par aspiration, et lavés par 2 ml de la solution de lavage. La radioactivité liée à chaque tube, proportionnelle à la quantité d'ACTH dans l'échantillon, est mesurée sur un compteur gamma pendant 1 minute. La concentration de chaque échantillon est calculée en rapportant la mesure de radioactivité effectuée sur la courbe standard tracée à l'aide des solutions d'ACTH standards.

Ce dosage possède la même sensibilité que les meilleurs kits commerciaux tels que, mais il est réalisé en 4 heures alors que ceux-ci réclament 20 heures d'incubation.

### Exemple 6 : Stabilité de l'ACTH succinylée

Des solutions calibrées d'ACTH dans du sérum humain ont été dosées suivant le protocole ci-dessus. Une partie de ces solutions est ensuite modifiée par l'anhydride succinique, puis conservée 24 heures à 20-25°C, et enfin dosée suivant le protocole ci-dessus ; l'autre partie est directement conservée à 20-25°C pendant 24 heures, puis modifiée par l'anhydride succinique, et enfin dosée suivant le même protocole. Les résultats de ces dosages montrent que, après conservation 24 heures à 20-25°C, on retrouve environ 61 % de l'ACTH modifiée, et seulement 2.7 % de l'ACTH native. La modification de l'ACTH en solution dans du sérum par l'anhydride succinique, entraîne donc une augmentation de la stabilité de la molécule.

L'homme de l'art appréciera qu'il est également possible de traiter un échantillon avec un agent réactif
- sur une fonction -COOH d'un reste acide aspartique ou glutamique ou
- sur une fonction -COOH terminale d'un peptide, ou encore
- sur une fonction -SH, d'un reste cystéine
pour effectuer le couplage d'un reste dudit agent réactif sur ledit antigène polypeptidique X et ainsi obtenir un antigène polypeptidique X modifié, (antigène polypeptidique Xm). Parmi les agents réactifs, on pourra donc citer également les réactifs d'alkylation des groupements -SH portés par les cystéines comme les haloacétates et les amides correspondantes tels que iodoacétate, bromoacétate, chloroacétate, iodoacétamides, bromoacétamide et chloroacétamide ou comme les dérivés maléimides tel que le N-éthylmaléimide, et citer enfin les carbodiimides, telles que la (1 éthyl 3 diméthylaminopropyl) carbodiimide (EDC) associées à un composé nucléophile approprié, tel que la glycinamide, qui permettent de modifier les groupements -COOH des acides glutamique et aspartique et les groupements -COOH terminaux des polypeptides.

On peut donc aussi utiliser un ou plusieurs agents réactifs de modification chimique pour modifier deux ou plus de deux types de groupement réactif.

## Revendications

1. Procédé d'identification ou de dosage d'un antigène polypeptidique X particulier dans lequel on procède à une détermination immunologique de la présence dudit antigène polypeptidique X dans un échantillon, caractérisé en ce que l'on traite in vitro l'échantillon à l'aide d'au moins un agent réactif
- sur une fonction epsilon -NH₂ d'un reste lysine ou
- sur une fonction alpha -NH₂ d'un peptide,
pour effectuer le couplage d'un reste dudit agent réactif sur ledit antigène polypeptidique X et ainsi obtenir un antigène polypeptidique X modifié, (antigène polypeptidique Xm),
puis procède à la détermination immunologique de la présence dudit antigène polypeptidique X dans ledit échantillon à l'aide d'au moins un anticorps spécifique dudit antigène polypeptidique Xm et non spécifique de la modification elle-même, produit par immunisation contre un antigène polypeptidique Y identique à l'antigène polypeptidique Xm ou à un fragment antigénique de cet antigène Xm.

2. Procédé selon la revendication 1 caractérisé en ce que l'on utilise un antigène polypeptidique Y identique à l'antigène polypeptidique Xm complet pour effectuer les immunisations.

3. Procédé selon la revendication 1 caractérisé en ce que l'on utilise un antigène polypeptidique Y correspondant à un fragment de l'antigène polypeptidique Xm pour effectuer l'immunisation et ainsi obtenir des anticorps dirigés contre une région particulière de l'antigène Xm.

4. Procédé selon l'une des revendications 1 à 3 caractérisé en ce que l'on immunise un animal avec l'antigène polypeptidique Y, ledit antigène étant couplé à une protéine porteuse.

5. Procédé selon l'une des revendications 1 à 4 caractérisé en ce que l'on utilise un seul agent réactif.

6. Procédé selon l'une des revendications 1 à 4, caractérisé en ce que l'on utilise plusieurs agents réactifs.

7. Procédé selon l'une des revendications 1 à 6 caractérisé en ce que le, ou les agents réactifs sont sélectionnés parmi : l'anhydride succinique, l'anhydride acétique, les dérivés des esters de N-hydroxy succinimide.

8. Procédé selon l'une des revendications 1 à 7 caractérisé en ce que l'échantillon est un fluide biologique tel que le sang, le sérum, l'urine, la sueur, les larmes.

9. Procédé selon l'une des revendications 1 à 8 caractérisé en ce que l'antigène polypeptidique X est l'hormone corticotrope humaine (ACTH).

10. Procédé selon l'une des revendications 1 à 8 caractérisé en ce que l'antigène polypeptidique est la parathormone humaine (PTH).

11. Procédé selon l'une des revendications 1 à 1**0** caractérisé en ce que la méthode de dosage est un dosage immunologique par compétition ou
un dosage radioimmunométrique, utilisant un traceur radioactif ou luminescent ou fluorescent ou enzymatique, ou
- un dosage immunologique par agglutination de particules,
- ladite méthode utilisant un ou plusieurs anticorps monoclonaux ou polyclonaux.

12. Procédé selon l'une des revendications 1 à **7** caractérisé en ce que l'échantillon est un prélèvement de tissus et l'antigène polypeptidique X est détecté par une méthode immunohistochimique.

13. Procédé selon l'une des revendications 1 à **7** caractérisé en ce que l'échantillon est une suspension cellulaire et où l'antigène polypeptidique X est détecté par une méthode de cytométrie en flux.

14. Un hybridome produisant des anticorps monoclonaux anti-ACTH acylée par l'anhydride succinique, choisi parmi les hybridomes déposés à la CNCM le 12 décembre 1995 sous les numéros I-1648, I-1649 et I-1650.

15. Un anticorps monoclonal anti-ACTH acylée produit par un hybridome tel que défini à la revendication 14.

## Patentansprüche

1. Verfahren zur Identifikation oder zur Messung eines bestimmten Polypeptid-Antigens X, bei welchem eine immunologische Bestimmung des Vorliegens dieses Polypeptid-Antigens X in einer Probe durchgeführt wird, dadurch gekennzeichnet, daß die Probe in vitro mit Hilfe zumindest eines Reagenz
- für eine ε-NH₂-Funktion eines Lysinrests oder
- für eine α-NH₂-Funktion eines Peptids
behandelt wird, um die Kopplung eines Rests des Reagenz mit dem Polypeptid-Antigen X vorzunehmen, und um so ein modifiziertes Polypeptid-Antigen X (Polypeptid-Antigen Xm) zu erhalten, dann die immunologische Bestimmung des Vorliegens des Polypeptid-Antigens X in der Probe mit Hilfe eines Antikörpers, der für das Polypeptid-Antigen Xm spezifisch und für die Modifikation selbst unspezifisch ist, durchgeführt wird, und der durch eine Immunisierung gegen ein Polypeptid-Antigen Y produziert wird, das mit dem Polypeptid-Antigen Xm oder mit einem Antigen-Fragment dieses Antigens Xm identisch ist.

2. Verfahren nach Anspruch 1, dadurch gekennzeichnet, daß ein Polypeptid-Antigen Y verwendet wird, das mit dem vollständigen Polypeptid-Antigen Xm identisch ist, um Immunisierungen vorzunehmen.

3. Verfahren nach Anspruch 1, dadurch gekennzeichnet, daß ein Polypeptid-Antigen Y verwendet wird, das einem Fragment des Polypeptid-Antigens Xm entspricht, um eine Immunisierung vorzunehmen, und so Antikörper gegen eine bestimmte Region des Antigens Xm zu erhalten.

4. Verfahren nach einem der Ansprüche 1 bis 3, dadurch gekennzeichnet, daß ein Tier mit dem Polypeptid-Antigen Y immunisiert wird, wobei das Antigen mit einem Trägerprotein gekoppelt wird.

5. Verfahren nach einem der Ansprüche 1 bis 4, dadurch gekennzeichnet, daß ein einziges Reagenz verwendet wird.

6. Verfahren nach einem der Ansprüche 1 bis 4, dadurch gekennzeichnet, daß mehrere Reagenzien verwendet werden.

7. Verfahren nach einem der Ansprüche 1 bis 6, dadurch gekennzeichnet, daß das oder die Reagenzien ausgewählt werden aus: Bernsteinsäureanhydrid, Essigsäureanhydrid, N-Hydroxysuccinimidester-Derivaten.

8. Verfahren nach einem der Ansprüche 1 bis 7, dadurch gekennzeichnet, daß die Probe eine biologische Flüssigkeit, wie Blut, Serum, Urin, Schweiß, Tränen, ist.

9. Verfahren nach einem der Ansprüche 1 bis 8, dadurch gekennzeichnet, daß das Polypeptid-Antigen X das humane corticotrope Hormon (ACTH) ist.

10. Verfahren nach einem der Ansprüche 1 bis 8, dadurch gekennzeichnet, daß das Polypeptid-Antigen das humane Parathormon (PTH) ist.

11. Verfahren nach einem der Ansprüche 1 bis 10, dadurch gekennzeichnet, daß das Meßverfahren eine immunologische Konkurrenzmessung oder
- eine radioimmunometrische Messung unter Verwendung eines radioaktiven oder lumineszenten oder fluoreszenten oder enzymatischen Trägers oder
- eine immunologische Messung durch Teilchenagglutination ist,
- bei welchem Verfahren ein oder mehrere monoklonale oder polyklonale Antikörper verwendet werden.

12. Verfahren nach einem der Ansprüche 1 bis 7, dadurch gekennzeichnet, daß die Probe entnommenes Gewebe umfaßt, und das Polypeptid-Antigen X durch ein immunohistochemisches Verfahren detektiert wird.

13. Verfahren nach einem der Ansprüche 1 bis 7, dadurch gekennzeichnet, daß die Probe eine Zellsuspension ist, und das Polypeptid-Antigen X durch ein Flußcytometrie-Verfahren detektiert wird.

14. Hybridom, welches monoklonale Antikörper gegen durch Bernsteinsäureanhydrid acyliertes ACTH produziert, ausgewählt aus den Hybridomen, die bei der CNCM am 12. Dezember 1995 unter den Nummern I-1648, I-1649 und I-1650 hinterlegt wurden.

15. Monoklonaler Antikörper gegen acyliertes ACTH, welcher durch ein wie in Anspruch 14 definiertes Hybridom produziert wird.

## Claims

1. Process for the identification or assay of a particular polypeptide antigen X in which an immunological determination is carried out for the presence of said polypeptide antigen X in a sample, characterized in that the sample is treated in vitro using at least one reagent
- on an epsilon -NH₂ function of a lysine residue or
- on an alpha -NH₂ function of a peptide,
in order to carry out the coupling of a remainder of said agent to said polypeptide antigen X and in this way to obtain a modified polypeptide antigen X, (polypeptide antigen Xm), the immunological determination of the presence of said polypeptide antigen X in said sample is then carried out using at least one antibody specific for said polypeptide antigen Xm and non-specific for the modification itself, produced by immunization against a polypeptide antigen Y identical to the polypeptide antigen Xm or to an antigenic fragment of this antigen Xm.

2. Process according to claim 1 characterized in that a polypeptide antigen Y identical to the complete polypeptide antigen Xm is used to carry out the immunizations.

3. Process according to claim 1 characterized in that a polypeptide antigen Y corresponding to a fragment of the polypeptide antigen Xm is used to carry out the immunization and in this way to obtain antibodies directed against a particular region of the antigen Xm.

4. Process according to one of claims 1 to 3 characterized in that an animal is immunized with the polypeptide antigen Y, said antigen being coupled with a carrier protein.

5. Process according to one of claims 1 to 4 characterized in that a single reagent is used.

6. Process according to one of claims 1 to 4 characterized in that several reagents are used.

7. Process according to one of claims 1 to 6 characterized in that the reagent or reagents are selected from: succinic anhydride, acetic anhydride, derivatives of the esters of N-hydroxy succinimide.

8. Process according to one of claims 1 to 7 characterized in that the sample is a biological fluid such as blood, serum, urine, sweat or tears.

9. Process according to one of claims 1 to 8 characterized in that the polypeptide antigen X is human corticotropic hormone (ACTH).

10. Process according to one of claims 1 to 8 characterized in that the polypeptide antigen is human parathyroid hormone (PTH).

11. Process according to one of claims 1 to 10 characterized in that the assay method is an immunoassay by competition or
- a radioimmunometric assay using a radioactive or luminescent or fluorescent or enzymatic tracer, or
- an immunoassay by particle agglutination,
said method using one or more monoclonal or polyclonal antibodies.

12. Process according to one of claims 1 to 7 characterized in that the sample is a tissue sample and the polypeptide antigen X is detected by an immunohistochemical method.

13. Process according to one of claims 1 to 7 characterized in that the sample is a cellular suspension and where the polypeptide antigen X is detected by a flow cytometry method.

14. A hybridoma producing antiacylated ACTH with succinic anhydride monoclonal antibodies, selected from the hybridomas deposited at the CNCM on 12^{th} December 1995 under the numbers I-1648, I-1649 and I-1650.

15. An anti-acylated ACTH monoclonal antibody produced by a hybridoma as defined in claim 14.
